# EUROPEAN PATENT SPECIFICATION

(11) **EP 1 380 574 B1**
(45) Date of publication and mention of the grant of the patent: **20.05.2009**
(21) Application number: 03077547.2
(22) Date of filing: 17.12.1999
(51) Int. Cl.: C07D 219/10

(54) **9-aminoacridine derivates and process for the preparation thereof**
9-Aminoacridin Derivate und Verfahren für ihre Herstellung
Derivés de la 9-aminoacridine et processus pur leur preparation

(30) Priority: 18.12.1998 KR 9856185
(43) Date of publication of application: 14.01.2004
(62) Divisional of application: 99959999.6
(73) Proprietor: Samjin Pharmaceutical Co., Ltd., Seoul 121-210 (KR)
(72) Inventor: Cho, Eui-Hwan, Kangnam-ku Seoul, 135-241 (KR); Chung, Sun-Gan, Kwonson-gu, Suwon Kyungki-do 441-460 (KR); Lee, Sun-Hwan, Kyungki-do 459-100 (KR); Kwon, Ho-Seok, Suwon, Kyungki-do 441-390 (KR); Kang, Dong-Wook, Dongan-gu,Anyang Kyungki-do 431-070 (KR); Joo, Jeong-Ho, Manan-gu, Anyang-si, Kyungki-do 430-042 (KR)
(74) Representative: Blokland, Arie

(56) References cited:
- WO-A-91/05770
- US-A- 4 575 553
- US-A- 5 354 864
- TSANN-LONG SU ET AL: "9-SUBSTITUTED ACRIDINE DERIVATIVES WITH LONG HALF-LIFE AND POTENT ANTITUMOR ACTIVITY: SYNTHESIS AND STRUCTURE-ACTIVITY RELATIONSHIPS" JOURNAL OF MEDICINAL CHEMISTRY, AMERICAN CHEMICAL SOCIETY. WASHINGTON, US, vol. 38, 23 October 1995 (1995-10-23), pages 3226-3235, XP000941839 ISSN: 0022-2623

## Description

The present invention relates to a new 9-aminoacridine derivative of the general formula (I) 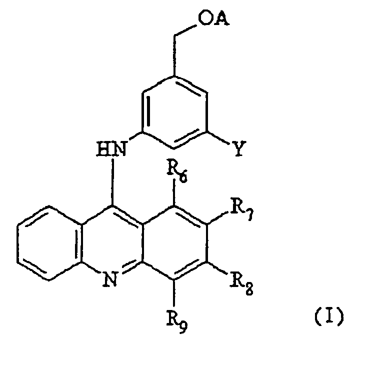 wherein A is hydrogen or 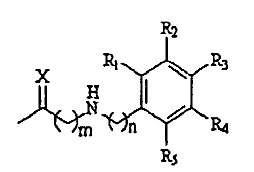 (wherein X is oxygen or sulfur, R₁, R₂, R₃, R₄ and R₅ are independently hydrogen, halogen, nitro, amino, hydroxy, C₁-C₄ lower alkylhydroxy, C₁-C₄ lower alkylamino, C₁-C₈ alkyl, C₁-C₄ lower alkoxy or C₁-C₄ lower alkyloxycarbonyl and m and n are independently an integer of 0, 1 or 2.),
R₆, R₇, R₈ and R₉ are independently hydrogen, C₁₋₈ alkyl or (C₁-C₄) lower alkoxy,
and Y is -N=CHR' (wherein R' is hydrogen, benzyl, C₁₋₈ alkyl of (C₁-C₆) lower alkylamino), 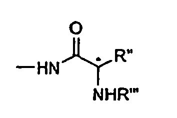 (wherein R'' is hydrogen, benzyl, C₁-C₈ alkyl or C₁-C₆ lower alkylamino, and R''' is hydrogen, benzyl, C₁-C₈ alkyl or amino protecting group) or 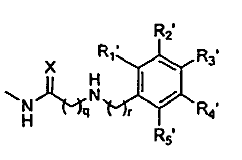 (wherein, X is as defined above, R₁', R₂', R₃', R₄' and R₅' are independently hydrogen, halogen, nitro, amino, hydroxy, C₁-C₄ lower alkylhydroxy, C₁-C₄ lower alkylamino, C₁-C₈ alkyl, C₁-C₄ lower alkoxy or C₁-C₄ lower alkyloxycarbonyl, and q and r are independently an integer of 0, 1 or 2) or its pharmaceutically acceptable salt, and process for the preparation thereof.

In the above compounds of the formula (I) wherein Y is 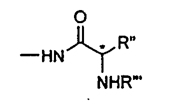 (R" and R''' are as defined above.), there may be isomers of *l*-form, *d*-form or racemic form.

In the above definitions, C₁-C₈ alkyl means straight or branched alkyl groups such as methyl, ethyl, propyl, isopropyl, *n*-butyl, isobutyl, *sec*-butyl, pentyl, isopentyl, hexyl, heptyl, octyl and 2-methylpentyl.
C₁-C₄ lower alkoxy means methoxy, ethoxy, propoxy, isopropoxy, butoxy, isobutoxy, tert-butoxy or the like.
C₁-C₄ lower alkylcarboxy means an esterified carboxy by a lower alkyl.
C₁-C₄ lower alkylamino means methylamino, ethylamino, propylamino, butylamino or the like.
C₁-C₄ lower alkylhydroxy means methylhydroxy, ethylhydroxy, propylhydroxy or the like.
Amino protecting group may include benzyl, benzyloxycarbonyl, t-butoxycarbonyl, 2,2,2-trichloroethoxycarbonyl, methoxycarbonyl and 2-methylsulfonylethoxycarbonyl.

WO-A-9105770, US-A-5354864, US-A-4575553 and Watanabe et al. (J. Med. Chem. 1995, 38, 3226-3235) describe structurally related anti-cancer agents.

The inventors had studied for a long time to find new compounds having intensive antitumor activities. As a result, the inventors have found out that the compounds of the general formula (I), or acid addition salts thereof as defined above have not only prominent antitumor activities but also very low toxicities.

Accordingly, an object of the invention is to provide a compound of the general formula (I) or acid addition salt thereof having not only prominent antitumor activity but also very low toxicity.
Another object of the invention is to provide a process for the preparation of the compound of the general formula (I) or acid addition salt thereof.

The compounds of the present invention can be mixed with pharmaceutically acceptable vehicles by a conventional method to give pharmaceutical preparations to be used for prevention or treatment of various kinds of tumors.

Therefore, the other object of the present invention is to provide pharmaceutical preparations containing an effective amount of a compound of the general formula (I) or acid addition salt thereof as an active ingredient.

Acids which can be reacted with the compound of the general formula(I) to form acid addition salt thereof are pharmaceutically acceptable inorganic acids, organic acids, amino acids or sulfonic acids; for example, inorganic acids such as hydrochloric acid, hydrobromic acid, sulfuric acid, phosphoric acid and nitric acid; organic acids such as formic acid, acetic acid, propionic acid, succinic acid, citric acid, maleic acid and malonic acid; amino acids such as serine, cysteine, cystine, asparagine, glutamine, lysine, arginine, tyrosine and proline; sulfonic acids such as methanesulfonic acid, ethanesulfonic acid, benzenesulfonic acid and toluenesulfonic acid.

Vehicles used in formulating pharmaceutical preparations containing the compound of the general formula(I) as an active ingredient are sweetening agents, binding agents, dissolving agents, aids for dissolution, wetting agents, emulsifying agents, isotonic agents, adsorbents, degrading agents, antioxidents, preservatives, lubricating agents, fillers, perfume or the like; for example may include lactose, dextrose, sucrose, mannitol, sorbitol, cellulose, glycine, silica, talc, stearic acid, stearin, magnesium stearate, calcium stearate, magnesium aluminum silicate, starch, gelatine, tragacanth gum, glycine, silica, alginic acid, sodium alginate, methyl cellulose, sodium carboxy methyl cellulose, agar, water, ethanol, polyethylenglycol, polyvinyl pyrrolidone, sodium chloride, potassium chloride, orange essence, strawberry essence and vanilla aroma.

Daily dosage of the compound of the general formula (I) may be varied depending on age, sex and degree of disease, but preferably 1mg to 5,000mg per day may be administered by once to several times.

The compound of the general formula (I) according to the present invention may be prepared by following schemes I, II or III. 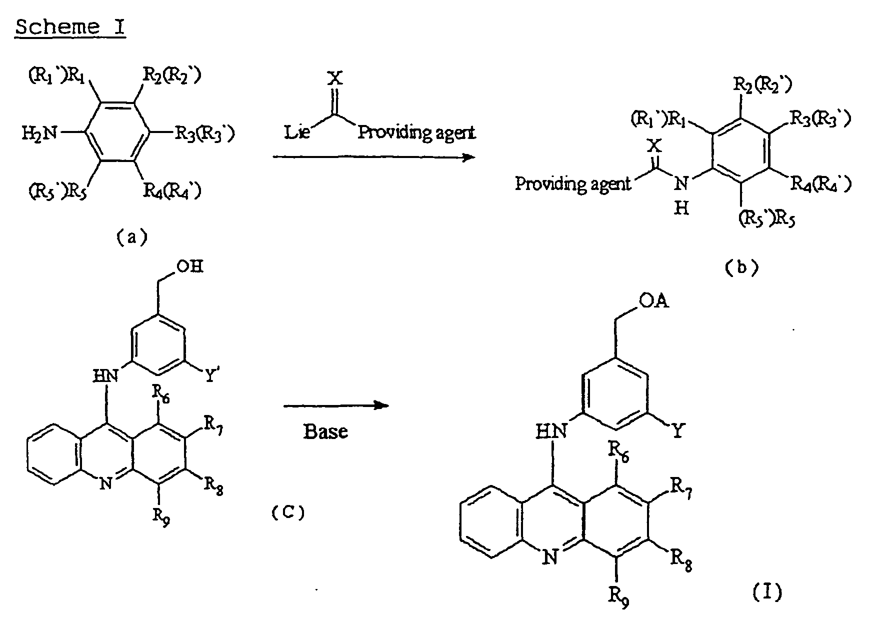 wherein R₁, R₂, R₃, R₄, R₅, R₆, R₇, R₈, R₉, R₁', R₂', R₃', R₄', R₅', A and Y are as defined above, Y' is H or NH₂, and Lie is a leaving group such as hydrogen and halogen atom.

According to the above scheme, a compound of the general formula (a) is reacted with a -C(=X)-group-providing agent in organic solvent to give a compound of the general formula(b), and successively the compound (b) is reacted with a compound of the general formula (c) to give a compound of the general formula (I).

The -C(=X)-group-providing agent used may include, for example, 1,1-carbonyldiimidazole, 1,1-carbonylthiodiimidazole, phosgene, thiophosgene, carbonyldiphenoxide and phenylchloroformate, and it may be used in an amount of 1-1.5 equivalent, preferably 1-1.1 equivalent to the starting compound.

The reaction may be carried out preferably in a conventional organic solvent such as tetrahydrofuran, dichloromethane, chloroform, acetonitrile and dimethylformamide.

In addition, the reaction may be carried out preferably in the presence of a coupling agent. Such coupling agent may include conventional inorganic or organic bases, for example, including sodium hydride, potassium hydride, sodium hydroxide, potassium hydroxide, sodium carbonate, potassium carbonate, cesium carbonate, sodium bicarbonate, potassium bicarbonate, triethylamine, pyridine and DBU, and it may be used by 1-5 equivalents.

The reaction may be carried out at a temperature between 3°C and boiling point of a used solvent, preferably at 50°C-100°C for 5-48 hours, preferably for 10-24 hours. 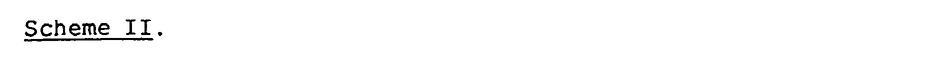 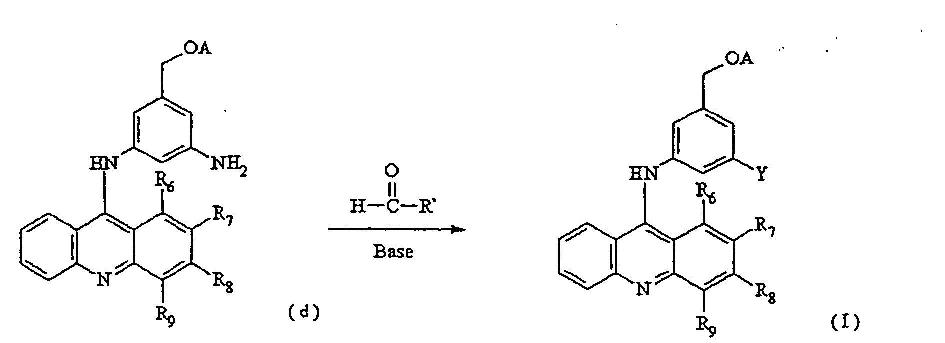 wherein, R₆, R₇, R₈, R₉, R', A and Y are as defined above.

According to the above scheme II, a compound of the general formula (d) above may be reacted with HCOR' in the presence of a base and a conventional organic solvent to give a compound of the general formula (I).

In the above reaction, the conventional organic solvent may include tetrahydrofuran, dichloromethane, chloroform, acetonitrile and dimethylformamide.

In addition, the reaction is carried out in the presence of a conventional inorganic or organic base as a coupling agent, and such a conventional base may include sodium hydride, potassium hydride, sodium hydroxide, potassium hydroxide, sodium carbonate, potassium carbonate, cesium carbonate, sodium bicarbonate, potassium bicarbonate, triethylamine, pyridine and DBU, and may be used in an amount of 1-5 equivalents.

The reaction may be carried out at a temperature between 3°C and acetonitrile and dimethylformamide.

And also the reaction is carried out preferably in the presence of a conventional organic or inorganic base as a coupling agent, and such a base may include sodium hydride, potassium hydride, sodium hydroxide, potassium hydroxide, sodium carbonate, potassium carbonate, cesium carbonate, sodium bicarbonate, potassium bicarbonate, triethylamine, pyridine and DBU, and it may be used in an amount of 1-5 equivalents.

The reaction may be carried out at a temperature between 3°C and boiling point of the solvent used, preferably at 50°C-100°C for 5-48 hours, preferably for 10-24 hours.

In the above processes according to the present invention, in case any acid material is formed, a suitable basic material may be added before reaction in order to eliminate the acid material from the reaction system. Such a basic material may be alkali metal hydroxide, alkali earth metal hydroxide, alkali metal oxide, alkali earth metal oxide, alkali metal carbonate, alkali earth metal carbonate, alkali metal hydrogen carbonate, alkali earth metal hydrogen carbonate such as sodium hydroxide, potassium hydroxide, calcium hydroxide, magnesium hydroxide, magnesium oxide, calcium oxide, potassium carbonate, sodium carbonate, calcium carbonate, magnesium carbonate, magnesium bicarbonate, sodium bicarbonate and calcium bicarbonate, or organic amines.

The compound of the general formula(c) is a known compound, boiling point of the solvent used, preferably at 50°C-100°C for 5-48 hours, preferably for 10-24 hours. 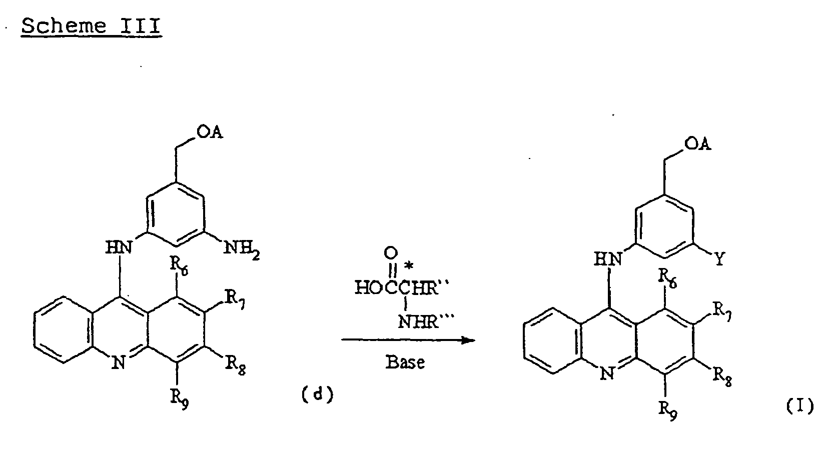 wherein, R₆, R₇, R₈, R₉, R'', R''', A and Y are as defined above.

According to the above scheme III, a compound of the general formula (d) may be reacted with a compound of following formula, 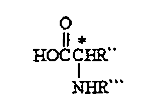 in the presence of a base and a conventional organic solvent to give a compound of the general formula (I).

The resulting compound of the formula (I) according to the scheme III may have isomers of *l*-form, *d*-form or racemic form.

In the above reaction, the conventional organic solvent may include tetrahydrofuran, dichloromethane, chloroform, described in, for example, J. Med. Chem., 1995, 38, 3226 or may be prepared in a similar method thereto.

### Examples

Compounds of the general formula (I) were prepared according to the above-mentioned processes of the invention.

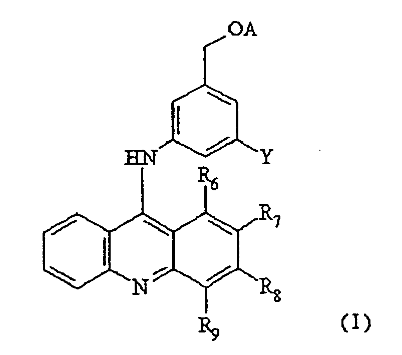

Reference-Examples 1-29 : A compound of the general formula (I) wherein

| 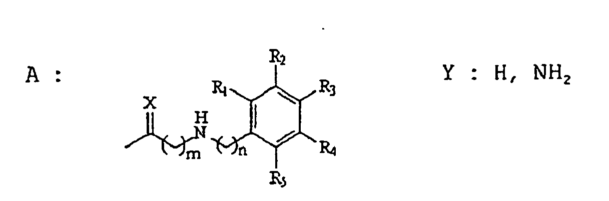 | | | | | | | | | | | | | |
|---|---|---|---|---|---|---|---|---|---|---|---|---|---|
| Reference-Ex. No. | X | m | n | R₁ | R₂ | R₃ | R₄ | R₅ | R₆ | R₇ | R₈ | R₉ | Y |
| 1 | O | 0 | 0 | H | H | H | H | H | H | H | H | H | NH₂ |
| 2 | O | 0 | 0 | H | OCH₃ | H | H | H | H | H | H | H | NH₂ |
| 3 | O | 0 | 0 | H | OCH₃ | H | OCH₃ | H | H | H | H | H | NH₂ |
| 4 | O | 0 | 0 | H | OCH₃ | OCH₃ | OCH₃ | H | H | H | H | H | NH₂ |
| 5 | O | 0 | 0 | H | H | CH₃ | H | H | H | H | H | H | NH₂ |
| 6 | O | 0 | 0 | H | CH₃ | H | CH₃ | H | H | H | H | H | NH₂ |
| 7 | O | 0 | 0 | H | F | H | H | H | H | H | H | H | NH₂ |
| 8 | O | 0 | 0 | H | H | F | H | H | H | H | H | H | NH₂ |
| 9 | O | 0 | 0 | F | H | H | F | H | H | H | H | H | NH₂ |
| 10 | O | 0 | 0 | F | H | F | H | H | H | H | H | H | NH₂ |
| 11 | O | 0 | 0 | H | F | F | H | H | H | H | H | H | NH₂ |
| 12 | O | 0 | 0 | H | F | H | F | H | H | H | H | H | NH₂ |
| 13 | O | 0 | 0 | Cl | H | H | H | H | H | H | H | H | NH₂ |
| 14 | O | 0 | 0 | H | Cl | H | H | H | H | H | H | H | NH₂ |
| 15 | O | 0 | 0 | H | Cl | H | Cl | H | H | H | H | H | NH₂ |
| 16 | O | 0 | 0 | H | OH | H | H | H | H | H | H | H | NH₂ |
| 17 | S | 0 | 0 | H | OCH₃ | H | H | H | H | H | H | H | NH₂ |
| 18 | S | 0 | 0 | H | OCH₃ | H | OCH₃ | H | H | H | H | H | NH₂ |
| 19 | S | 0 | 0 | H | OCH₃ | OCH₃ | OCH₃ | H | H | H | H | H | NH₂ |
| 20 | O | 0 | 0 | H | OCH₃ | H | H | H | H | H | H | H | H |
| 21 | O | 0 | 0 | H | OCH₃ | H | OCH₃ | H | H | H | H | H | H |
| 22 | O | 0 | 0 | H | OCH₃ | OCH₃ | OCH₃ | H | H | H | H | H | H |
| 23 | O | 0 | 0 | H | F | H | F | H | H | H | CH₃ | CH₃ | NH₂ |
| 24 | O | 0 | 0 | H | Cl | H | Cl | H | H | H | CH₃ | CH₃ | NH₂ |
| 25 | O | 0 | 0 | H | Cl | H | Cl | H | H | CH₃ | H | H | NH₂ |
| 26 | O | 0 | 0 | H | F | H | F | H | H | CH₃ | H | H | NH₂ |
| 27 | O | 0 | 0 | H | F | H | F | H | H | CH₂ | CH₃ | H | NH₂ |
| 28 | O | 0 | 0 | H | F | H | F | H | H | H | H | OCH₃ | NH₂ |
| 29 | O | 0 | 0 | H | Cl | H | Cl | H | H | H | H | OCH₃ | NH₂ |

Examples 30-34 : A compound of the general formula (I) wherein

| 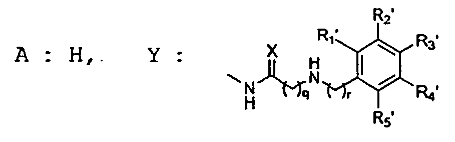 | | | | | | | | | | | | | |
|---|---|---|---|---|---|---|---|---|---|---|---|---|---|
| (q, r = integer) | | | | | | | | | | | | | |
| Ex. No. | X | q | r | R₁' | R₂' | R₃' | R₄' | R₅' | R₆ | R₇ | R₈ | R₉ | A |
| 30 | O | 0 | 0 | H | F | H | F | H | H | H | H | H | H |
| 31 | O | 0 | 0 | H | F | H | H | H | H | H | H | H | H |
| 32 | O | 0 | 0 | H | Cl | H | Cl | H | H | H | H | H | H |
| 33 | O | 0 | 0 | H | F | H | F | H | H | H | H | OCH₃ | H |
| 34 | O | 0 | 0 | H | F | H | F | H | H | H | CH₃ | CH₃ | H |

Examples 35-37 : A compound of the general formula (I) wherein

| A : H, Y : N=CHR', R | | | | | | |
|---|---|---|---|---|---|---|
| Ex. No. | A | R' | R₆ | R₇ | R₈ | R₉ |
| 35 | H | CH₃ | H | H | H | H |
| 36 | H | CH₂CH₃ | H | H | H | H |
| 37 | H | CH₂CH₂CH₃ | H | H | H | H |

Examples 38∼53 : A compound of the general formula (I) wherein

| 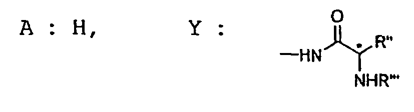 | | | | | | | |
|---|---|---|---|---|---|---|---|
| Ex. No. | A | R'' | R''' | R₆ | R₇ | R₈ | R₉ |
| 38 | H | H | 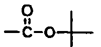 | H | H | H | H |
| 39 | H | CH₃ | // | H | H | H | H |
| 40 | H | CH₂Ph | // | H | H | H | H |
| 41 | H | CH(CH₃)₂ | // | H | H | H | H |
| 42 | H | CH₂CH(CH)₂ | // | H | H | H | H |
| 43 | H | CH(CH₃)CH₂CH₃ | // | H | H | H | H |
| 44 | H | H | H | H | H | H | H |
| 45 | H | CH₃ | H | H | H | H | H |
| 46 | H | CH₂Ph | H | H | H | H | H |
| 47 | H | CH(CH₃)₂ | H | H | H | H | H |
| 48 | H | CH₂CH(CH₃)₂ | H | H | H | H | H |
| 49 | H | CH(CH₃)CH₂CH₃ | H | H | H | H | H |
| 50 | H | CH₃ | H | H | CH₃ | H | H |
| 51 | H | CH₃ | H | H | H | CH₃ | CH₃ |
| 52 | H | CH₃ | H | H | . H | H | OCH₃ |
| 53 | H | CH₂CH₂CH₂CH₂NH₂ | H | H | H | H | H |

### Reference-Example 1 : [3-(acridin-9-yl)amino-5-amino]benzyl N-phenylcarbamate

### a) Phenyl N-carbamate

Triethylamine(1.11g, 11.0mmol) was added to a solution of aniline(1.00g, 11.0mmol) dissolved in dichloromethane(20ml), and phenylchloroformate (1.76g, 11.0mmol) was added thereto by dropping. The resulting solution was stirred for 2 hours at the room temperature, washed with distilled water, concentrated and purified by column chromatography to give the titled compound.
Yield : 81.7%
m.p. : 119∼120°C
¹H NMR(CDCl₃) :δ 5.10(1H,brs), 6.63(1H,dd), 6.67(2H,d), 7.05(1H,m), 7.13(2H,m), 7.46(2H,m), 7.60(2H,m)

### b) [3-(acridin-9-yl)amino-5-amino]benzyl N-phenylcarbamate

3-(9-acridinylamino)-5-(hydroxymethyl)aniline(1.00g, 3.17mmol) and phenyl N-phenylcarbamate(0.67g, 3.17mmol) were dissolved in dimethylformaldehyde(40ml), and thereto DBU(0.49g, 3.17mmol) was added by dropping. After stirring the resulting mixture for 12 hours at the room temperature, the used solvent was removed under the reduced pressure. The resulting product was purified by column chromatography to give the titled compound.
Yield : 62.5%
m.p. : 98∼100°C
¹H NMR(DMSO-d₆) : δ 5.03(2H,s), 6.13(1H,s), 6.35(2H,d), 6.99(1H,t), 7.25(2H,t), 7.46(9H,brs), 7.55(2H,brs), 8.09(1H, brs)

### Reference-Example 2 : [3-(acridin-9-yl)amino-5-amino]benzyl N-(3-methoxyphenyl)carbamate

3-(9-acridinylamino)-5-(hydroxymethyl)aniline and phenyl N-(3-methoxylphenyl) carbamate were reacted by the same method to the example 1 to give the titled compound.
Yield : 57.4%
m.p. : 83∼86°C
¹H NMR(DMSO-d₆) : δ 3.74(3H,s), 5.01(2H,s), 6.11(1H,s), 6.14(1H,s), 6.35(2H,s), 6.73(2H,s), 7.55(3H,m), 8.45(1H,s)

### Reference-Example 3 : [3-(acridin-9-yl)amino-5-amino]benzyl N-(3,5-dimethoxyphenyl)carbamate

3-(9-acridinylamino)-5-(hydroxymethyl)aniline and phenyl N-(3,5-dimethoxyphenyl)carbamate were reacted with the same method to the example 1 to give the titled compound.
Yield : 52.7%
m.p. 85∼88°C
¹H NMR(DMSO-d₆) : δ 3.75(6H,s), 5.02(2H,s), 6.11(1H,s), 6.13(1H,s), 6.35(2H,s), 6.73(2H,s), 7.56(3H,m), 8.44(1H,s)

### Reference-Example 4 : [3-(acridin-9-yl)amino-5-amino]benzyl N-(3,4,5-trimethoxyphenyl)carbamate

3-(9-acridinylamino)-5-(hydroxymethyl)aniline and phenyl N-(3,4,5-trimethoxyphenyl)carbamate were reacted with the same method to the example 1 to give the titled compound.
Yield : 47.6%
m.p. : 120~122°C
¹H NMR(DMSO-d₆) : δ 3.71(3H,s), 3.79(6H,s), 5.00(2H,s), 6.08(1H,s), 6.23(1H,s), 6.32(1H,s), 6.85(2H,s), 7.12(2H,brs), 7.48(4H,brs), 8.17(2H,brs), 9.31(1H,s), 10.50 (1H,brs)

### Reference-Example 5 : [3-(acridin-9-yl)amino-5-amino]benzyl N-(4-methylpheny)carbamate

3-(9-acridinylamino)-5-(hydroxymethyl)aniline and phenyl N-(4-methylphenyl) carbamate were reacted with the same method to the example 1 to give the titled compound.
Yield : 54.9%
m.p. : 95∼97°C
¹H NMR(DMSO-d₆) : δ 2.62(3H,s), 5.08(2H,s), 5.94(1H,s), 6.08(1H,brs), 6.26(1H,s), 7.10(3H,d), 7.37(4H,d), 7.52(2H,brs), 8.23(3H,brs), 9.62(1H,s), 10.8(1H,brs)

### Reference-Example 6 : [3-(acridin-9-yl)amino-5-amino]benzyl N-(3,5-dimethylphenyl)carbamate

3-(9-acridinylamino)-5-(hydroxymethyl)aniline and phenyl N-(3,5-dimethylphenyl)carbamate were reacted with the same method to the example 1 to give the titled compound.
Yield : 52.8%
m.p. : 118∼121°C
¹H NMR(DMSO-d₆) : δ 2.24(6H,s), 4.98(2H,s), 6.05(1H,s), 6.22(1H,s), 6.32(1H,s), 6.60(1H,s), 7.09(4H,brs), 7.47(4H,brs), 8.17(2H,brs), 9.24(1H,s), 10.5(1H,brs)

### Reference-Example 7 : [3-(acridin-9-yl)amino-5-amino]benzyl N-(3-fluorophenyl)carbamate

3-(9-acridinylamino)-5-(hydroxymethyl)aniline and phenyl N-(3-fluorophenyl) carbamate were reacted with the same method to the example 1 to give the titled compound.
Yield : 49.3%
m.p. : 110∼112°C
¹H NMR(DMSO-d₆) : 6 5.02(2H,s), 6.17(1H,s), 6.29(1H,s), 6.39(1H,s), 6.67(1H,brs), 7.09(2H,brs), 7.19(2H,s), 7.39(1H,d), 7.54(3H,brs), 7.63(1H,brs), 8.08(2H,brs), 9.60(1H,s)

### Reference-Example 8 : [3-(acridin-9-yl)amino-5-amino]benzyl N-(4-fluorophenyl)carbamate

3-(9-acridinylamino)-5-(hydroxymethyl)aniline and phenyl N-(4-fluorophenyl)carbamate were reacted with the same method to the example 1 to give the titled compound.
Yield : 48.9%
m.p. : 161∼163°C
¹H NMR(DMSO-d₆) : δ 5.02(2H,s), 6.13(1H,s), 6.33(2H,d), 6.93(2H,t), 7.11(2H,brs), 7.45(2H,brs), 7.53(2H,brs), 7.68(2H,brs), 8.07(2H,brs), 8.98(1H,brs)

### Reference-Example 9 : [3-(acridin-9-yl)amino-5-amino]benzyl N-(2,5-difluorophenyl)carbamate

3-(9-acridinylamino)-5-(hydroxymethyl)aniline and phenyl N-(2,5-difluorophenyl)carbamate were reacted with the same method to the example 1 to give the titled compound.
Yield : 46.8%
m.p. : 188∼193°C
¹H NMR(DMSO-d₆) : δ 5.06(2H,s), 6.23(1H,s), 6.38(1H,s), 6.42(1H,s), 6.68(1H,m), 7.01(1H,m), 7.15(2H,brs), 7.57(2H,t), 7.82(3H,brs), 8.03(1H,s), 8.10(2H,d), 8.17(1H,brs)

### Reference-Example 10 : [3-(acridin-9-yl)amino-5-amino]benzyl N-(2,4-difluoropheny)carbamate

3-(9-acridinylamino)-5-(hydroxymethyl)aniline and phenyl N-(2,4-difluorophenyl)carbamate were reacted with the same method to the example 1 to give the titled compound.
Yield : 47.0%
m.p. : 100∼102°C
¹H NMR(DMSO-d₆) : δ 5.03(2H,s), 6.17(1H,s), 6.32(1H,s), 6.39(1H,s), 6.85(2H,m), 7.10(2H,brs), 7.54(2H,brs), 7.66(3H,brs), 7.78(1H,brs), 8.08(2H,brs), 8.54(1H,brs)

### Reference-Example 11 : [3-(acridin-9-yl)amino-5-amino]benzyl N-(3,4-difluorophenyl)carbamate

3-(9-acridinylamino)-5-(hydroxymethyl)aniline and phenyl N-(3,4-difluorophenyl)carbamate were reacted with the same method to the example 1 to give the titled compound.
Yield : 46.8%
m.p. : 123∼125°C
¹H NMR(DMSO-d₆) : δ 5.01(2H,s), 6.13(1H,s), 6.31(2H,s), 7.08(4H,m), 7.37(1H,s), 7.54(3H,brs), 7.71(1H,brs), 8.05(2H,brs), 8.86(1H,brs)

### Reference-Example 12 : [3-(acridin-9-yl)amino-5-amino]benzyl N-(3,5-difluorophenyl)carbamate

3-(9-acridinylamino)-5-(hydroxymethyl)aniline and phenyl N-(3,5-difluorophenyl)carbamate were reacted with the same method to the example 1 to give the titled compound.
Yield : 46.5%
m.p. : 125∼128°C
¹H NMR(DMSO-d₆) : δ 5.01(2H,s), 6.13(1H,s), 6.27(1H,s), 6.32(1H,s), 6.41(1H,t), 7.06(2H,brs), 7.13(3H,d), 7.50(3H,t), 7.60(3H,brs), 8.05(3H,brs), 9.67(1H,s)

### Reference-Example 13 : [3-(acridin-9-yl)amino-5-amino]benzyl N-(2-chlorophenyl)carbamate

3-(9-acridinylamino)-5-(hydroxymethyl)aniline and phenyl N-(2-chlorophenyl) carbamate were reacted with the same method to the example 1 to give the titled compound.
Yield : 42.0%
m.p. : 162∼164°C
¹H NMR(DMSO-d₆) : δ 5.03(2H,s), 6.34(4H,s), 6.97(1H,d), 7.12(2H,t), 7.29(2H,d), 7.55(2H,brs), 7.62(2H,s), 8.02(2H,brs), 8.80(1H,s)

### Reference Example 14 : [3-(acridin-9-yl)amino-5-amino]benzyl N-(3-chlorophenyl)carbamate

3-(9-acridinylamino)-5-(hydroxymethyl)aniline and phenyl N-(3-chlorophenyl) carbamate were reacted with the same method to the example 1 to give the titled compound.
Yield : 46.8%
m.p. : 135∼137°C
¹H NMR(DMSO-d₆) : δ 5.03(2H,s), 6.13(1H,s), 6.34(3H,s), 6.97(1H,d), 7.17(2H,t), 7.29(2H,d), 7.55(2H,brs), 7.62(2H,s), 8.08(2H,brs), 8.80(1H,s)

### Reference-Example 15 : [3-(acridin-9-yl)amino-5-amino]benzyl N-(3,5-dichlorophenyl)carbamate

3-(9-acridinylamino)-5-(hydroxymethyl)aniline and phenyl N-(3,5-dichlorophenyl)carbamate were reacted with the same method to the example 1 to give the titled compound.
Yield : 44.2%
m.p. : 188∼190°C
¹H NMR(DMSO-d₆) : δ 5.06(2H,s), 6.68(1H,s), 6.93(1H,s), 7.16(2H,brs), 7.45(2H,s), 7.67(4H,brs), 7.94(2H,s), 8.13(2H,brs), 8.82(1H,brs), 9.03(1H,brs)

### Reference-Example 16 : [3-(acridin-9-yl)amino-5-amino]benzyl N-(3-hydroxyphenyl)carbamate

3-(9-acridinylamino)-5-(hydroxymethyl)aniline and phenyl N-(3-hydroxyphenyl) carbamate were reacted with the same method to the example 1 to give the titled compound.
Yield : 37.8%
m.p. : 152∼153°C
¹H NMR(DMSO-d₆) : δ 4.98(1H,s), 5.08(2H,s), 6.27(1H,s), 6.42(1H,d), 6.88(1H,d), 7.06(4H,m), 7.31(2H,brs), 7.48(4H,brs), 8.21(2H,brs), 9.35(1H,s), 9.61(1H,s), 10.82(1H,brs)

### Reference-Example 17 : [3-(acridin-9-yl)amino-5-amino]benzyl N-(3-methoxyphenyl)thiocarbamate

### a) Phenyl N-(3-methoxyphenyl)thiocarbamate

Triethylamine(0.87g, 8.63mmol) was added to a solution of 3-methoxyaniline(1g, 8.63mmol) dissolved in dichloromethane(20ml), and thereto phenylchlorothioformate(1.49g, 8.63mmol) was added by dropping. The resulting solution was stirred for 2 hours at the room temperature, washed with distilled water, concentrated and purified by column chromatography to give the titled compound.
Yield : 77.4%
m.p. : 166∼168°C
¹H NMR(CDCl₃) : δ 5.11(1H,brs), 6.61(1H,dd), 6.64(2H,d), 7.11(3H,m), 7.20(2H,m), 7.35(2H,m)

### b) [3-(acridin-9-yl)amino-5-amino]benzyl N-(3-methoxyphenyl)thiocarbamate

3-(9-acridinylamino)-5-(hydroxymethyl)aniline(0.75g, 2.38mmol) and phenyl N-(3-methoxyphenyl)thiocarbamate(0.62g, 2.38mmol) were dissolved in dimethylformaldehyde(40ml), and thereto DBU(0.36g, 2.38mmol) was added by dropping. The resulting solution was stirred for 12 hours at the room temperature, and the solvent used was removed under the reduced pressure. Then, column chromatography was carried out to give the titled compound.
Yield : 59.4%
m.p. : 163∼165°C
¹H NMR(DMSO-d₆) : δ 3.76(3H,s), 5.01(2H,s), 6.66(2H,s), 6.95(2H,m), 7.10(1H,s), 7.17(1H,s), 7.22(1H,s), 7.52(6H,brs), 8.00(2H,d), 9.39(1H,s), 9.55(1H,s), 10.8(1H,brs)

### Reference-Example 18 : [3-(acridin-9-yl)amino-5-amino]benzyl N-(3,5-dimethoxyphenyl)thiocarbamate

3-(9-acridinylamino)-5-(hydroxymethyl)aniline and phenyl N-(3,5-dimethoxyphenyl)thiocarbamate were reacted with the same method to the example 17 to give the titled compound..
Yield : 51.7%
m.p. : 158∼160°C
¹H NMR(DMSO-d₆) : δ 3.74(3H,s), 5.44(2H,s), 6.23(1H,s), 6.74(2H,s), 6.83(1H,s), 7.22(4H,m), 7.65(4H,m), 8.15(2H,brs), 9.41(1H,brs), 9.55(1H,brs)

### Example 19 : [3-(acridin-9-yl)amino-5-amino]benzyl N-(3,4,5-trimethoxyphenyl)thiocarbamate

3-(9-acridinylamino)-5-(hydroxymethyl)aniline and phenyl N-(3,4,5-trimethoxyphenyl)thiocarbamate were reacted with the same method to the example 17 to give the titled compound..
Yield : 47.9%
m.p. : 148∼150°C
¹H NMR(DMSO-d₆) : δ 3.60(3H,s), 3.74(6H,s), 5.44(2H,s), 6.25(1H,s), 6.73(2H,s), 6.83(1H,s), 7.21(4H,m), 7.75(4H,m), 8.15(2H,brs), 9.41(1H,brs), 9.55(1H,brs)

### Reference-Example 20 : 3-(acridin-9-yl)aminobenzyl N-(3-methoxyphenyl)carbamate

[3-(9-acridinylamino)phenyl]methanol(1.37g, 4.56mmol) and phenyl N-(3-methoxyphenyl)carbamate(1.11g, 4.56mmol) were dissolved in dimethylformaldehyde(40ml) and thereto DBU(0.69g, 4.56mmol) was added by dropping. The resulting mixture was stirred for 6 hours at the room temperature and the solvent used was removed under the reduced pressure. Then, the resulting product was purified by column chromatography to give the titled compound. Yield : 70.4%
m.p. : 77∼78°C
¹H NMR(DMSO-d₆) : δ 3.78(3H,s), 5.12(2H,s), 6.16(1H,d), 6.84(3H,t), 7.00(2H,m), 7.11(1H,brs), 7.18(1H,t), 7.24(2H,m), 7.58(2H,t), 7.92(2H,brs), 7.99(2H,d)

### Reference-Example 21 : 3-(acridin-9-yl)aminobenzyl N-(3,5-dimethoxyphenyl)carbamate

3-(9-acridinylamino)phenylmethanol and phenyl N-(3,5-dimethoxyphenyl) carbamate were reacted with the same method to the example 20 to give the titled compound..
Yield : 68.9%
m.p. : 108∼110°C
¹H NMR(DMSO-d₆) : δ 3.76(6H,s), 5.13(2H,s), 6.19(1H,s), 6.60(2H,s), 6.73(1H,brs), 6.86(1H,brs), 7.02(2H,m), 7.25(6H,brs), 7.59(2H,brs), 7.99(2H,brs)

### Reference-Example 22 : 3-(acridin-9-yl)aminobenzyl N-(3,4,5-trimethoxyphenyl)carbamate

3-(9-acridinylamino)phenylmethanol and phenyl N-(3,4,5-trimethoxyphenyl) carbamate were reacted with the same method to the example 20 to give the titled compound..
Yield : 67.9%
m.p. : 94∼96°C
¹H NMR(DMSO-d₆) : δ 3.59(3H,s), 3.70(6H,s), 5.12(2H,s), 6.69(2H,d), 6.83(3H,d), 7.01(2H,d), 7.32(3H,m), 7.45(2H,brs), 8.11(1H,brs), 9.64(1H,brs), 10.90(1H,s)

### Reference-Example 23 : [3-(3,4-dimethylacridin-9-yl)amino-5-amino]benzyl N-(3,5-difluorophenyl)carbamate

3-(3,4-dimethylacridin-9-yl)amino-5-(hydroxymethyl)aniline and phenyl N-(3,5-difluorophenyl)carbamate were reacted with the same method to the example 1 to give the titled compound..
Yield : 51.6%.
m.p. : 190∼191°C
¹H NMR(DMSO-d₆) : δ 2.49(3H,s), 2.83(3H,s), 5.02(2H,s), 5.13(1H,s), 5.81(1H,s), 6.15(2H.d), 6.88(2H,m), 7.18(1H,m), 7.30(1H,d), 7.45(1H,m), 7.73(1H,m), 7.94(1H,d), 8.11(1H,d), 8.16(1H,d), 8.85(1H,s), 10.17(1H,s)

### Reference-Example 24 : [3-(3,4-dimethylacridin-9-yl)amino-5-amino]benzyl N-(3,5-dichlorophenyl)carbamate

3-(3,4-dimethylacridin-9-yl)amino-5-(hydroxymethyl)aniline and phenyl N-(3,5-dichlorophenyl)carbamate were reacted with the same method to the example 1 to give the titled compound..
Yield : 52.5%
m.p. : 136∼138°C
¹H NMR(DMSO-d₆) : δ 2.47(3H,s), 2.81(3H,s), 4.97(2H,s), 5.06(1H,s), 5.92(1H,s), 6.14(2H.m), 7.24(2H,m), 7.42(1H,m), 7.53(2H,s), 7.71(1H,m), 7.94(1H,m), 8.15(2H,m), 8.88(1H,s), 10.16(1H,s)

### Reference-Example 25 : [3-(2-methylacridin-9-yl)amino-5-amino]benzyl N-(3,5-dichlorophenyl)carbamate

3-(2-methylacridin-9-yl)amino-5-(hydroxymethyl)aniline and phenyl N-(3,5-dichlorophenyl)carbamate were reacted with the same method to the example 1 to give the titled compound..
Yield : 53.7%
m.p. : 208∼209°C
¹H NMR(DMSO-d₆) : δ 2.35(3H,s), 5.01(2H,s), 5.15(1H,s), 6.02(1H,s), 6.14(1H,s), 6.29(1H.s), 7.25(2H,m), 7.53(4H,m), 7.61(2H,m), 7.96(2H,m), 10.16(1H,s)

### Reference-Example 26 : [3-(2-methylacridin-9-yl)amino-5-amino]benzyl N-(3,5-difluorophenyl)carbamate

3-(2-methylacridin-9-yl)amino-5-(hydroxymethyl)aniline and phenyl N-(3,5-difluorophenyl)carbamate were reacted with the same method to the example 1 to give the titled compound..
Yield : 56.5%
m.p. : 170∼172°C
¹H NMR(DMSO-d₆) : δ 2.37(3H,s), 5.03(2H,s), 5.18(1H,s), 6.14(1H,s), 6.24(1H,s), 6.37(1H.s), 6.88(2H,m), 7.17(4H,m), 7.68(2H,m), 8.03(2H,m), 10.19(1H,s)

### Reference-Example 27 : [3-(2,3-dimethylacridin-9-yl)amino-5-amino]benzyl N-(3,5-difluorophenyl)carbamate

3-(2,3-dimethylacridin-9-yl)amino-5-(hydroxymethyl)aniline and phenyl N-(3,5-difluorophenyl)carbamate were reacted with the same method to the example 1 to give the titled compound..
Yield : 51.2%
m.p. : 140∼142°C
¹H NMR(DMSO-d₆) : δ 2.25(3H,s), 2.39(3H,s), 5.02(2H,s), 5.18(1H,s), 6.07(1H,s), 6.19(1H.s), 6.33(1H,s), 6.87(1H,m), 7.17(3H,m), 7.65(4H,m), 8.05(1H,m), 10.18(1H,s)

### Reference-Example 28 : [3-(4-methoxyacridin-9-yl)amino-5-amino]benzyl N-(3,5-difluorophenyl)carbamate

3-(4-methoxyacridin-9-yl)amino-5-(hydroxymethyl)aniline and phenyl N-(3,5-difluorophenyl)carbamate were reacted with the same method to the example 1 to give the titled compound..
Yield : 57.5%
m.p. 178∼180°C
¹H NMR(DMSO-d₆) : δ 4.03(3H,s), 5.01(2H,s), 5.10(1H,s), 5.96(1H,s), 6.07(1H,s), 6.26(1H.s), 6.87(1H,m), 7.19(4H,m), 7.50(2H,m), 7.75(2H,m), 8.15(1H,m), 10.21(1H,s), 10.27(1H,s)

### Reference-Example 29 : [3-(4-methoxyacridin-9-yl)amino-5-amino]benzyl N-(3,5-dichlorophenyl)carbamate

3-(4-methoxyacridin-9-yl)amino-5-(hydroxymethyl)aniline and phenyl N-(3,5-dichlorophenyl)carbamate were reacted with the same method to the example 1 to give the titled compound..
Yield : 58.6%
m.p. : 230∼232°C
¹H NMR(DMSO-d₆) : δ 4.02(3H,s), 5.02(2H,s), 5.09(1H,s), 5.95(1H,s), 6.03(1H,s), 6.26(1H.s), 7.11(2H,m), 7.24(1H,s), 7.54(4H,m), 7.72(2H,m), 8.16(1H,m), 10.18(1H,s), 10.24(1H,s)

### Example 30 : N-[3-(acridin-9-yl)amino-5-hydroxymethyl]phenyl N'-(3,5-difluorophenyl)urea

### a) Phenyl N-(3,5-difluorophenyl)carbamate

Triethylamine(1.11g, 11.0mmol) was added to a solution of 3,5-difluoroaniline(1.42g, 11.0mmol) dissolved in dichloromethane (20ml), and thereto phenylchloroformate(1.76g, 11.2mmol) was added by dropping. The resulting mixture was stirred for 2 hours at the room temperature, washed with distilled water, concentrated and purified by column chromatography to give the titled compound.
Yield : 86.5%
m.p. : 126∼128°C
¹H NMR(CDCl₃) :δ 6.84(3H,s), 7.30(5H,d)

### b) N-[3-(acridin-9-yl)amino-5-hydroxymethyl]phenyl N'-(3,5-difluorophenyl)urea

3-(9-acridinylamino)-5-(hydroxymethyl)aniline(1.00g, 3.17mmol) and phenyl N-(3,5-difluorophenyl)carbamate(0.79g, 3.17mmol) were dissolved in dimethylformaldehyde(40ml), and thereto DBU(0.48g, 3.17mmol) was added by dropping. The resulting solution was stirred for 2 hours at the room temperature and the solvent used was removed under the reduced pressure. Then, purification by column chromatography was carried out to give the titled compound.
Yield : 21.0%
m.p. : 125∼128°C
¹H NMR(DMSO-d₆) : δ 4.70(2H,s), 5.04(2H,s), 6.36(1H,s), 6.41(2H,m), 6.54(1H,s), 7.13(2H,d), 7.20(2H,brs), 7.67(2H,brs), 7.74(2H,brs), 8.18(2H,brs), 9.79(1H,s)

### Example 31 : N-[3-(acridin-9-yl)amino-5-hydroxymethyl]phenyl N'-(3-fluorophenyl)urea

3-(9-acridinylamino)-5-(hydroxymethyl)aniline and phenyl N-(3-fluorophenyl) carbamate were reacted with the same method to the example 30 to give the titled compound..
Yield : 20.2%
m.p. : 292°C (decomposed)
¹H NMR(DMSO-d₆) : δ 4.57(2H,s), 7.24(1H,s), 7.27(2H,d), 7.47(2H,m), 7.85(5H,s), 8.00(2H,m), 8.21(2H,d), 8.37(2H,d), 11.5(1H,s)

### Example 32 : N-[3-(acridin-9-yl)amino-5-hydroxymethyl]phenyl N'-(3,5-dichlorophenyl)urea

3-(9-acridinylamino)-5-(hydroxymethyl)aniline and phenyl N-(3,5-dichlorophenyl)carbamate were reacted with the same method to the example 30 to give the titled compound..
Yield : 18.5%
m.p. : 180∼181°C
¹H NMR(DMSO-d₆) : 6 4.52(2H,s), 5.06(1H,s), 6.68(1H,s), 6.93(1H,s), 7.18(3H,m), 7.45(2H,s), 7.67(4H,m), 7.94(2H,s), 8.13(2H,m), 8.82(1H,s), 9.03(1H,s)

### Example 33 : N-[3-(4-methoxyacridin-9-yl)amino-5-hydroxymethyl]phenyl N'-(3,5-difluorophenyl)urea

3-(4-methoxyacridin-9-yl)amino-5-(hydroxymethyl)aniline and phenyl N-(3,5-difluorophenyl)carbamate were reacted with the same method to the example 30 to give the titled compound..
Yield : 20.5%
m.p. : 164∼165°C
¹H NMR(DMSO-d₆) : δ 4.03(3H,s), 4.52(2H,s), 6.36(1H,s), 6.78(2H,m), 7.03(3H,m), 7.11(2H,m), 7.13(4H,m), 8.19(2H,m), 8.69(1H,s), 10.93(1H,s)

### Example 34 : N-[3-(3,4-dimethylacridin-9-yl)amino-5-hydroxymethyl]phenyl N'-(3,5-difluorophenyl)urea

3-(9-acridinylamino)-5-(hydroxymethyl)aniline and phenyl N-(3,5-difluorophenyl)carbamate were reacted with the same method to the example 30 to give the titled compound..
Yield : 20.2%
m.p. : 185∼186°C
¹H NMR(DMSO-d₆) : δ 2.49(2H,s), 2.84(3H,s), 4.37(2H,s), 5.11(1H,s), 6.47(1H,s), 6.70(1H,s), 6.79(1H,m), 7.01(1H,s), 7.15(2H,m), 7.35(1H,m), 7.48(1H,m), 7.79(1H,m), 7.95(1H,m), 8.16(2H,m), 8.71(1H,s), 8.96(1H,s), 9.06(1H,s)

### Example 35 : 3-(acridin-9-yl)amino-5-(ethylideneamino)phenylmethanol

3-(9-acridinylamino)-5-(hydroxymethyl)aniline was dissolved in dichloromethane/pyridine(1/1, v/v, 40ml), and thereto acetaldehyde(2.09g, 47.56mmol) was added. The resulting mixture was stirred for 5 hours, and the solvent used was removed under the reduced pressure. Then, the resulting product was purified by column chromatography to give the titled compound.
Yield : 80.1%
m.p. : 158∼161°C
¹H NMR(DMSO-d₆) : δ 2.63(3H,s), 5.00(2H,s), 5.16(1H,brs), 7.11(2H,d), 7.17(1H,d), 7.35(1H,s), 7.60(2H,brs), 7.76(4H,brs), 8.10(2H,brs), 8.30(1H,d).

### Example 36 : 3-(acridin-9-yl)amino-5-(propylideneamino)phenylmethanol

3-(9-acridinylamino)-5-(hydroxymethyl)aniline and propylaldehyde were reacted with the same method to the example 35 to give the titled compound..
Yield : 78.9%
m.p. 262∼263°C
¹H NMR(DMSO-d₆) : δ 1.28(3H,t), 2.88(2H,m), 4.98(2H,s), 5.06(1H,brs), 5.42(1H,s), 7.09(3H,s), 7.28(1H,s), 7.56(3H,s), 7.73(1H,s), 8.80(3H,s)

### Example 37 : 3-(acridin-9-yl)amino-5-(butylideneamino)phenylmethanol

3-(9-acridinylamino)-5-(hydroxymethyl)aniline and butylaldehyde were reacted with the same method to the example 35 to give the titled compound..
Yield : 77.5%
m.p. : 228∼230°C
¹H NMR(DMSO-d₆) : δ 1.25(3H,t), 2.44(2H,s), 2.86(2H,m), 4.90(2H,d), 5.35(1H,s), 7.00(2H,d), 7.33(2H,d), 7.48(3H,brs), 7.82(1H,brs), 7.98(2H,brs), 8.08(1H,brs), 8.18(1H,brs), 9.43(1H,s), 10.97(1H,s)

### Example 38 : t-butyl

### N-{2-[3-(acridin-9-yl)amino-5-(hydroxymethyl)aniline]-2-oxoethyl}carbamate

3-(9-acridinylamino)-5-(hydroxymethyl)aniline(1.00g, 3.17mmol) was dissolved in pyridine(30ml), and thereto WSCD(0.62g, 3.17mmol), HOBT (0.43g, 3.17mmol) and 2-[(t-butoxycarbonyl)amino] acetic acid (0.56g, 3.17mmol) were added. The resulting solution was stirred for 10 hours at 0°C, and the solvent used was removed under the reduced pressure. Then, the resulting product was purified by column chromatography to give the titled compound.
Yield : 78.8%
m.p. : 193∼195°C
¹H NMR(DMSO-d₆) : 6 1.43(9H,s), 3.81(2H,s), 4.51(2H,s), 5.13(1H,s), 6.56(1H,s), 6.85(1H,s), 7.27(2H,s), 7.37∼ 7.54(2H,m), 7.64(1H,d), 7.98(2H,brs), 7.78(1H,s), 7.79(2H,s), 8.19(1H,s), 9.87(1H,s)

### Example 39 : t-butyl N-{2-[3-(acridin-9-yl)amino-5-(hydroxymethyl)anilino]-1-methyl-2-oxoethyl}carbamate

3-(9-acridinylamino)-5-(hydroxymethyl)aniline and t-butoxycarbonyl-L-alanine were reacted with the same method to the example 38 to give the titled compound.
Yield : 77.2%
m.p. : 131∼133°C
¹H NMR(DMSO-d₆) : δ 1.33(3H,d), 1.42(9H,s), 4.23(1H,m), 4.53(2H,d), 5.09(1H,brs), 6.41(1H,d), 6.81(1H,brs), 7.27(2H,brs), 7.42(1H,s), 7.76(2H,brs), 7.84(1H,brs), 8.19(1H,brs), 9.83(1H,brs)

### Example 40 : t-butyl N-{2-[3-(acridin-9-yl)amino-5-(hydroxymethyl)anilino]-(2S)-1-benzyl-2-oxoethyl}carbamate

3-(9-acridinylamino)-5-(hydroxymethyl)aniline and t-butoxycarbonyl-L-phenylalanine were reacted with the same method to the example 38 to give the titled compound.
Yield : 68.7%
m.p. : 193∼195°C
¹H NMR(DMSO-d₆) : δ 1.35(9H,s), 2.89(2H,m), 3.09(1H,m), 4.51(2H,s), 6.01(1H,s), 7.02(1H,s), 7.07(1H,brs), 7.16(1H,brs), 7.22(5H,s), 7.28(1H,s), 7.54(2H,m), 7.59(1H,brs), 8.03(2H,brs), 9.63(1H,s)

### Example 41 : t-butyl N-{2-[3-(acridin-9-yl)amino-5-(hydroxymethyl)anilino]-(2S)-1-isopropyl-2-oxoethyl}carbamate

3-(9-acridinylamino)-5-(hydroxymethyl)aniline and t-butoxycarbonyl-L-valine were reacted with the same method to the example 38 to give the titled compound.
Yield : 72.8%
m.p. : 179∼180°C
¹H NMR(DMSO-d₆) : δ 1.32(6H,t), 1.42(9H,s), 2.36(1H,m), 4.01(1H,d), 4.74(2H,s), 7.29(1H,s), 7.46(2H,t), 7.63(1H,s), 7.76(1H,s), 7.78(4H,m), 8.23(2H,d)

### Example 42 : t-butyl N-{2-[3-(acridin-9-yl)amino-5-(hydroxymethyl)aniline]-(2S)-1-(2-isobutyl)-2-oxoethyl}carbamate

3-(9-acridinylamino)-5-(hydroxymethyl)aniline and t-butoxycarbonyl-L-leucine were reacted with the same method to the example 38 to give the titled compound.
Yield : 68.7%
m.p. : 262∼263°C
¹H NMR(DMSO-d₆) : δ 0.94(6H,t), 1.42(2H,m), 1.46(9H,s), 1.75(1H,m), 3.35(1H,m), 4.73(2H,s), 7.19(1H,s), 7.47(2H,t), 7.74(1H,s), 7.85(1H,s), 7.80(4H,m), 8.35(2H,d)

### Example 43 : t-butyl N-{2-[3-(acridin-9-yl)amino-5-(hydroxymethyl)anilino]-(2S)-1-sec-butyl-2-oxoethyl}carbamate

3-(9-acridinylamino)-5-(hydroxymethyl)aniline and t-butoxycarbonyl-L-isoleucine were reacted with the same method to the example 38 to give the titled compound.
Yield : 61.9%
m.p. : 280∼282°C
¹H NMR(DMSO-d₆) : δ 0.90(3H,t), 0.96(3H,d), 1.29(2H,m), 1.43(9H,s), 1.98(1H,m), 3.40(1H,d), 4.73(2H,s), 7.29(1H,s), 7.56(2H,t), 7.74(1H,s), 7.85(1H,s), 7.90(4H,m), 8.25(2H,d)

### Example 44 : N-[3-(acridin-9-yl)amino-5-(hydroxymethyl)phenyl] aminoethaneamide

Anisole(0.47g, 4.28mmol) and acetonitrile/dichloromethane(1/2, v/v, 25ml) were added to t-butyl N-{2-[3-(9-acridinylamino)-5-(hydroxymethyl)anilino]-2-oxoethyl}carbamate(0.34g, 0.71mmol). To the resulting mixture, aluminium chloride (0.57g, 4.28mmol) was slowly added with stirring and stirred for 2 hours at the room temperature. The resulting product was concentrated under the reduced pressure and purified by column chromatography to give the titled compound.
Yield : 62.7%
m.p. : 360°C (decomposed)
¹H NMR(DMSO-d₆) : δ 3.87(2H,s), 4.63(2H,s), 7.19(1H,s), 7.45(2H,m), 7.64(1H,s), 7.72(1H,s), 7.99(4H,d), 8.24(2H,d)

### Example 45 : N-[3-(acridin-9-yl)amino-5-(hydroxymethyl)phenyl] (2S)-2-aminopropaneamide

The same reaction procedure to the example 44 was carried out using t-butyl N-{2-[3-(9-acridinylamino)-5-(hydroxymethyl)anilino]-1-methyl-2-oxoethyl}carbamate to give the titled compound.
Yield : 59.6%
m.p. : 289∼291°C
¹H NMR(DMSO-d₆) : δ 1.57(3H,d), 4.09(1H,q), 4.63(2H,s), 7.19(1H,s), 7.46(2H,t), 7.64(1H,s), 7.75(1H,s), 7.99(4H,m), 8.23(2H,d)

### Example 46 : N-[3-(acridin-9-yl)amino-5-(hydroxymethyl)phenyl] (2S)-2-amino-3-phenylpropaneamide

The same reaction procedure to the example 44 was carried out using t-butyl N-{2-[3-(9-acridinylamino)-5-(hydroxymethyl)anilino]-1-benzyl-2-oxoethyl}carbamate to give the titled compound.
Yield : 54.9%
m.p. : 246∼249°C
¹H NMR(DMSO-d₆) : δ 2.89(2H,m), 3.09(1H,m), 4.51(2H,s), 6.01(1H,s), 7.02(1H,s), 7.07(1H,brs), 7.16(1H,brs), 7.25(5H,s), 7.28(1H,s), 7.54(2H,m), 7.59(1H,brs), 8.03(2H,brs), 9.63(1H,s)

### Example 47 : N-[3-(acridin-9-yl)amino-5-(hydroxymethyl)phenyl] (2S)-2-amino-3-methylbutaneamide

The same reaction procedure to the example 44 was carried out using t-butyl N-{2-[3-(9-acridinylamino)-5-(hydroxymethyl)anilino]-1-isopropyl-2-oxoethyl}carbamate to give the titled compound.
Yield : 53.7%
m.p. : 181∼183°C
¹H NMR(DMSO-d₆) : δ 0.99(6H,s), 2.16(1H,m), 3.74(1H,s), 4.48(2H,s), 5.23(1H,s), 6.53(1H,s), 7.00(2H,m), 7.21(1H,m), 7.38(2H,m), 7.52(2H,m), 8.15(4H,m), 10.45(1H,s), 11.03(1H,s)

### Example 48 : N-[3-(acridin-9-yl)amino-5-(hydroxymethyl)phenyl] (2S)-2-amino-4-methylpentaneamide

The same reaction procedure to the example 44 was carried out using t-butyl N-{2-[3-(9-acridinylamino)-5-(hydroxymethyl)anilino]-1-(2-isobutyl)-2-oxoethyl}carbamate to give the titled compound.
Yield : 48.5%
m.p. : 220∼223°C
¹H NMR(DMSO-d₆) : δ 0.96(6H,d), 1.69(3H,m), 4.04(1H,s), 4.51(2H,s), 5.41(1H,s), 7.00(1H,s), 7.50(4H,m), 7.96(4H,m), 8.26(1H,m), 8.39(2H,m), 10.98(1H,s)

### Example 49 : N-[3-(acridin-9-yl)amino-5-(hydroxymethyl)phenyl] (2S)-2-amino-3-methylpentaneamide

The same reaction procedure to the example 44 was carried out using t-butyl N-{2-[3-(9-acridinylamino)-5-(hydroxymethyl)anilino]-1-sec-butyl-2-oxoethyl}carbamate to give the titled compound.
Yield : 46.8%
m.p. : 177∼179°C
¹H NMR(DMSO-d₆) : δ 0.90(3H,m), 0.96(3H,m), 1.18(1H,m), 1.59(1H,m), 1.90(1H,m), 3.73(1H,s), 4.49(1H,s), 5.24(1H,s), 6.52(1H,s), 7.13(2H,m), 7.36(2H,m), 7.51(2H,m), 8.17(4H,m), 10.34(1H,s), 10.99(1H,s)

### Example 50 : N-[3-(2-methylacridin-9-yl)amino-5-(hydroxymethyl)phenyl] (2S)-2-aminopropaneamide

The same reaction procedure to the example 44 was carried out using t-butyl N-{2-[3-(2-methylacridin-9-yl)amino-5-(hydroxymethyl)anilino]-1-methyl-2-oxoethyl}carbamate to give the titled compound.
Yield : 61.5%
m.p. : 280°C (decomposed)
¹H NMR(DMSO-d₆) : δ 1.50(3H,d), 2.45(3H,s), 4.16(1H,s), 4.49(2H,s), 5.44(1H,s), 7.05(1H.s), 7.41(1H,m), 7.65(1H,s), 7.72(1H,s), 7.97(2H,m), 8.18(2H,m), 8.33(1H,s), 8.44(2H,s), 11.21(1H,brs)

### Example 51

### N-[3-(3,4-dimethylacridin-9-yl)amino-5-(hydroxymethyl)phenyl] (2S)-2-aminopropaneamide

The same reaction procedure to the example 44 was carried out using t-butyl N-{2-[3-(3,4-dimethylacridin-9-yl)amino-5-(hydroxymethyl)anilino]-1-methyl-2-oxoethyl}carbamate to give the titled compound.
Yield : 62.4% %
m.p. : 238∼240°C
¹H NMR(DMSO-d₆) : δ 1.47(3H,d), 2.52(3H,s), 2.76(3H,s), 4.11(1H,s), 4.45(2H,s), 5.37(1H.s), 6.90(1H,s), 7.33(1H,m), 7.44(2H,m), 7.92(1H,s), 8.01(1H,s), 8.26(1H,s), 8.43(3H,m), 11.21(1H,brs)

### Example 52 : N-[3-(4-methoxyacridin-9-yl)amino-5-(hydroxymethyl)phenyl] (2S)-2-aminopropaneamide

The same reaction procedure to the example 44 was carried out using t-butyl N-{2-[3-(4-methoxyacridin-9-yl)amino-5-(hydroxymethyl)anilino]-1-methyl-2-oxoethyl}carbamate to give the titled compound.
Yield : 60.2%
m.p. : 260°C (decomposed)
¹H NMR(DMSO-d₆) : δ 1.51(3H,d), 3.19(1H,s), 4.19(3H,s), 4.48(2H,s), 7.06(1H,s), 7.42(1H,m), 7.43(1H.m), 7.58(1H,d), 7.75(2H,d), 7.93(1H,d), 8.01(1H,m), 8.38(1H,d), 8.47(1H,d), 8.55(1H,s), 11.44(1H,brs)

### Example 53 : N-[3-(acridin-9-yl)amino-5-(hydroxymethyl)phenyl] (2S)-2-amino-6-aminohexaneamide

The same reaction procedure to the example 44 was carried out using t-butyl N-{2-[3-(acridin-9-yl)amino-5-(hydroxymethyl)anilino]-1-methyl-2-oxoethyl}carbamate to give the titled compound.
Yield : 48.5%
m.p. : 281°C(decomposed)
¹H NMR(DMSO-d₆) : δ 1.47(2H,m), 1.65(2H,m), 1.89(2H,m), 2.77(2H,m), 4.14(2H,m), 4.48(2H,s), 5.42(1H.s), 6.97(1H,m), 7.41(2H,m), 7.60(2H,m), 7.93(2H,m), 8.20(4H,m), 8.56(2H,s), 11.32(1H,s)

The compounds prepared in the examples according to the present invention were tested for pharmacological activities against tumors. Antitumor activities of the compounds were tested *in vitro* against 5 kinds of human tumor cell lines and 2 kinds of leukemia tumor cell lines. In addition, inhibition effects of the compounds against the DNA Topoisomerase were measured by using DNA relaxation assay and DNA cleavage assay. Methods and results of the tests are as follows.

Experimental 1 : *In vitro* antitumor effect against human tumor cell lines.

### A. Tumor cell lines : A549 (human non-small lung cell)

SKOV-3 (human ovarian)
HCT-15 (human colon)
XF-498 (human CNS)
SKMEL-2 (human melanoma)

### B. Method : SRB Assay

a. Human solid tumor cell lines, A549(non-small lung cell), SKMEL-2(melanoma), HCT-15(colon), SKOV-3(ovarian) and XF-498(CNS) were cultured in 5% CO₂ incubators using the RPMI 1640 media containing 10% FBS at 37°C, while with transfer-culturing successively once or twice per week. Cell cultures were dissolved in a solution of 0.25% trysin and 3 mmol CDTA PBS(-) to separate the cells sticked on the culture media.
b. 5× 10³∼2× 10⁴ cells were added into each well of 96-well plate and cultured in 5% CO₂ incubator at 37°C for 24 hours.
c. Each sample drug was dissolved in a little DMSO and diluted with the used medium to a prescribed concentration for experiment, while the final concentration of DMSO was adjusted below 0.5%.
d. Medium of each well cultured for 24 hours as above b. was removed by aspiration. Each 200µl of drug samples prepared in c. was added into each well and the wells were cultured for 48 hours. Tz (time zero) plates were collected at the point of time drugs were added.
e. According to the SRB assay method, cell fixing with TCA, staining with 0.4% SRB solution, washing with 1% acetic acid and elution of dye with 10mmol Tris solution were carried out on Tz plates and culture-ended plates, and then, OD values were measured at 520 nm.

### C. Calculation of result

a. Time zero(Tz) value was determined with measuring the SRB protein value at the point of time drugs were added.
b. Control value (C) was determined with the OD value of an well untreated with drug.
c. Drug-treated test value (T) was determined with the OD value of drug-treated well.
d. Effects of drugs were estimated with growth stimulation, net growth inhibition and net killing calculated from Tz, C and T values.
e. If T ≥ Tz, cellular response function was calculated by 100x(T-Tz)/(C-Tz), and, if T 〈 Tz, by 100× (T-Tz)/Tz. The results are shown in the next table 1.

### * REFERENCE

1) P. Skehan, R. Strong, D Scudiero, A. Monks, J. B. Mcmahan, D. T. Vistica, J. Warren, H. Bokesh, S. Kenney and M. R. Boyd : Proc. Am. Assoc. Cancer Res., 30, 612 (1989).
2) L. V. Rubinstein, R. H. Shoemaker, K. D. Paull, R. M. Simon, S. Tosini, P. Skehan, D. Scudiero, A. Monks and M. R. Boyd ; J. Natl. Cancer Inst., 82, 1113 (1990).
3) P. Skehan, R. Strong, D. Scudiero, A. Monks, J. B. Mcmahan, D. T. Vistica, J. Warren, H. Bokesch, S. Kenney and M. R. Boyd. ; J, Natl. Cancer Inst., 82, 1107 (1990).

### D. Results.

It was found that the compounds of the present invention have the even or superior antitumor activities than that of cisplatin, the control against human solid cancer cell lines.

**Table 1.**

| ED₅₀=µ *g*/*ml* | | | | | |
|---|---|---|---|---|---|
| Comp. (No. of ex.) | A 549 | SK-OV-3 | SK-MEL-2 | XF-498 | HCT 15 |
| 1* | 0.96 | 1.15 | 0.49 | 0.44 | 0.57 |
| 2* | 1.12 | 0.68 | 1.08 | 0.71 | 0.97 |
| 4* | 0.19 | 0.19 | 0.20 | 0.23 | 0.24 |
| 6* | 0.72 | 0.36 | 0.40 | 0.37 | 0.33 |
| 37 | 0.90 | 0.93 | 0.89 | 0.36 | 0.35 |
| 45 | 0.01 | 0.21 | 0.25 | 0.88 | 0.86 |
| Cisplatin | 0.81 | 0.71 | 0.71 | 0.77 | 3.03 |

| | | | | | |
|---|---|---|---|---|---|
| * Reference examples | | | | | |

### Experimental 2 : In vitro antitumor effects against animal leukemia cells.

### A. Material :

Tumor cell lines : P388 (mouse lymphoid neoplasma cell)

### B. Method : Dye Exclusion Assay.

1) The concentration of P388 cells being cultured in RPMI 1640 media containing 10% FBS was adjusted to 1× 10⁶ cells/ml.
2) Each sample drug of a concentration diluted in the ratio of log dose was added into cell culture media and cultured at 37°C for 48 hours in 50% CO₂ incubator, and then viable cell number was measured by dye exclusion test using trypan blue.
3) The concentration of each sample compound showing 50 % cell growth inhibition(IC₅₀) compared with the control was determined and listed in the table 2 below.

### * REFERENCE

1) P. Skehan, R. Strong, D. Scudiero, A. Monks, J. B. Mcmahan, D. T. Vistica, J. Warren, H. Bokesch, S. Kenney and M. R. Boyd. : Proc. Am. Assoc. Cancer Res., 30, 612 (1989).
2) L. V. Rubinstein, R. H. Shoemaker, K. D. Paull, R. M. Simon, S. Tosini, P. Skehan, D. Scudiero, A. Monks and M. R. Boyd. : J. Natl. Cancer Inst., 82, 1113 (1990)
3) P. Skehan, R. Strong, D. Scudiero, J. B. Mcmahan, D. T. Vistica, J. Warren, H. Bokesch, S. KenneyandM. R. Boyd. : J. Natl. Cancer Inst., 82, 1107(1990)

### C. Results

As the result of measurement of antitumor activities against P388 mouse cancer cells of the compounds according to the present invention, it was found that the compounds tested have equal to or higher antitumor activities than those of the control drug, mitomycin C.

**Table 2**

| Comp. (No. of example) | P388 |
|---|---|
| 1 | 0.9 |
| 3 | 1.1 |
| 6 | 0.8 |
| 12 | 1.2 |
| 14 | 1.0 |
| 17 | 1.0 |
| 18 | 0.7 |
| 44 | 0.1 |
| 45 | 0.05 |
| 46 | 0.1 |
| Mitomycin C | 1.1 |

Experimental 3 : Inhibition effects against the Topoisomerase II activities

### a) DNA relaxation assay.

The present compounds were tested for inhibition of Topoisomerase II activity by means of the relaxation assay using supercoiled pBR322 DNA as the substrate. Topo II and a sample inhibitor were added to a reaction system (50mmol Tris-HCl, pH 7.5, 50mmol KCl, 20mmol MgCl₂, 0.5mmol EDTA, 2mmol ATP, 60µg/mℓ BSA) containing supercoiled pBR322 DNA and reacted for 30 minutes at 37°C. Then, a quarter by volume of Stop buffer (5% SDS, 50mmol EDTA, 30% glycerol, 0.1mg/mℓ xylene cyanol, 0.1mg/mℓ BPB) was added to the reaction system to stop the reaction. The resulting product went through electrophoresis on 0.7% agarose gel and was stained by ethidium bromide solution. Mobility of DNA was measured under ultraviolet rays.

### b) DNA cleavage assay

Topo II and a sample inhibitor were added to cleavage buffer (30mmol Tris-HCl, pH 7.5, 60mmol KCl, 10mmol MgCl₂, 15mmol β -mercaptoethanol, 30µg/mℓ BSA) containing supercoiled pBR322 DNA and allowed to react for 30 minutes at 37°C. Then, SDA by 1% was added to the reaction system to stop the reaction. Then, proteinase K was added thereto up to the concentration of 50 µg/mℓ and allowed to react for 30 minutes at 56°C. The reaction product was treated with phenol/chloroform to purify DNA, and the obtained DNA was loaded on agarose gel electrophoresis, transferred to nitrocellulose membrane and hybridized with probe DNA labelled with radioisotope. Then, the nitrocellulose membrane was covered with an X-ray film and exposed to be sensitized and developed, whereby the degree of cleavage of DNA was measured.

### c) Results

As the result of inhibition tests against Topoisomerase II activity as shown in the following table, it was found that the compounds according to the present invention have the superior antitumor activity than the control, etoposide.

**Table 3**

| Comp. (No. of | IC₅₀(µg/mℓ) | Comp. (No. of | IC₅₀(µg/mℓ) |
|---|---|---|---|
| 1 | 1 | 21 | 3 |
| 2 | 3 | 22 | 5 |
| 3 | 3 | 35 | 5 |
| 4 | 5 | 36 | 3 |
| 6 | 3 | 37 | 5 |
| 12 | 0.5 | 44 | 3 |
| 14 | 1 | 45 | 0.5 |
| 18 | 3 | 46 | 3 |
| 19 | 5 | Etoposide | 10 |
| 20 | 3 | | |

### Experimental 4. Acute toxicity test (LD₅₀) :

### a) Method : Litchfield-Wilcoxon method.

6-week-old ICR mice (male 30± 2.0g) were fed freely with solid feed and water at room temperature, 23± 1°C and at humidity 60 ± 5%. Sample drugs were injected into the abdominal cavities of mice. Each group comprised 6 mice. Observed during 14 days, external appearances and life or death thereof were recorded, and also, visible lesions were observed from dead mice by dissection. LD₅₀ value was calculated by Litchfield-wilcoxon method.

### b) Results

As shown in the following table, the compounds according to the present invention are predominantly safe in comparison with cisplatin, whereby much problems of known compounds such as restriction of dosage, unfavorable side effects by toxicity, etc. may be overcome considerably.

**Table 4**

| Comp. (No. of example) | LD₅₀ (mg/kg) | |
|---|---|---|
| | i.p. | i.v. |
| 12 | 150 | |
| 45 | 730 | 133 |
| Cisplatin | 9.7 | |

As described above, the compounds according to the present invention are much more safer and also have much superior antitumor activities to known anticancer drugs, and accordingly the compounds are expected to be useful as a new anticancer agent.

## Claims

1. A compound of the general formula(I) 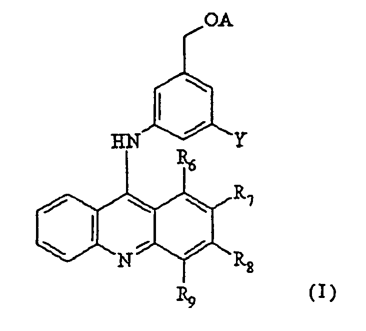 wherein A is hydrogen or 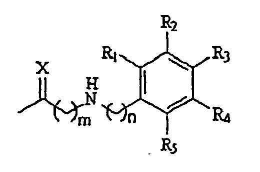 (wherein X is oxygen or sulfur, R₁, R₂, R₃, R₄ and R₅ are independently hydrogen, halogen, nitro, amino, hydroxy, C₁-C₄ lower alkylhydroxy, C₁-C₄ lower alkylamino, C₁-C₈ alkyl, C₁-C₄ lower alkoxy or C₁-C₄ lower alkyloxycarbonyl and m and n are independently an integer of 0, 1 or 2.),
R₆, R₇, R₈ and R₉ are independently hydrogen, C₁₋₈ alkyl or (C₁-C₄) lower alkoxy,
and Y is -N=CHR_{'} (wherein R' is hydrogen, benzyl, C₁₋₈ alkyl of (C₁-C₆) lower alkylamino), 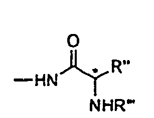 (wherein R'' is hydrogen, benzyl, C₁-C₈ alkyl or C₁-C₆ lower alkylamino, and R''' is hydrogen, benzyl, C₁-C₈ alkyl or amino protecting group) or 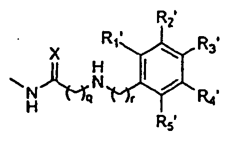 wherein, X is as defined above,
R₁', R₂', R₃', R₄' and R₅' are independently hydrogen, halogen, nitro, amino, hydroxy, C₁-C₄ lower alkylhydroxy, C₁-C₄ lower alkylamino, C₁-C₈ alkyl, C₁-C₄ lower alkoxy or C₁-C₄ lower alkylcarboxy, and q and r are independently an integer of 0, 1 or 2) or its pharmaceutically acceptable salt.

2. A process for the preparation of a compound according to claim 1, comprising reacting a compound of the formula (a) with a -C=X-providing reagent to give a compound of the formula (b), reacting the compound of the formula (b) with a compound of the formula (c) to give a compound according to claim 1, and optionally converting it to an acid addition salt thereof. 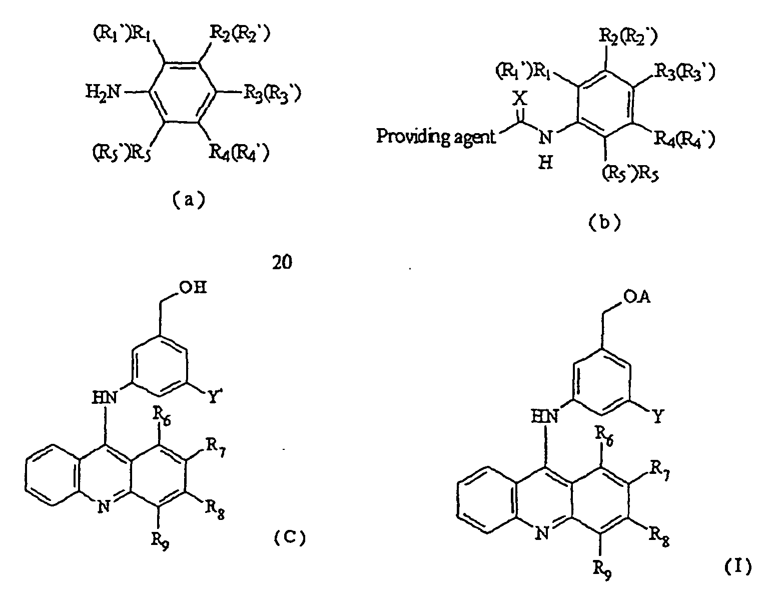 wherein Y' is H or -NH₂, and the other substituents are as defined in claim 1.

3. A process for the preparation of a compound according to claim 1, comprising reacting a compound of the general formula (d) with HCOR' in the presence of a base and an organic solvent to give a compound of formula (I), 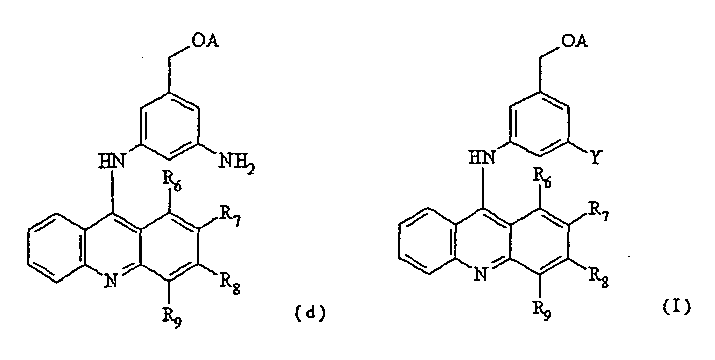 wherein the substituents are as defined in claim 1.

4. A process for the preparation of a compound according to claim 1 comprising reacting a compound of the general formula (d) with 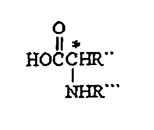 in the presence of a base and an organic solvent to give a compound of formula (I), 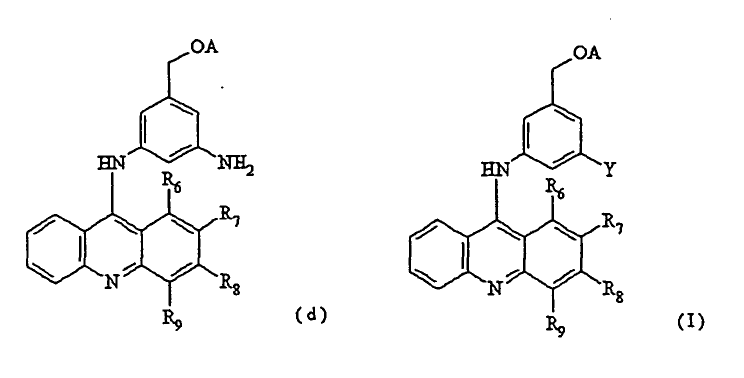 wherein the substituents are as defined in claim 1.

## Patentansprüche

1. Verbindung der allgemeinen Formel (1) 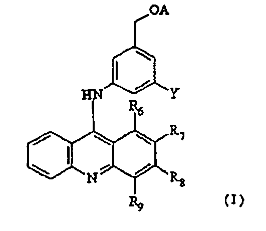 wobei A Wasserstoff ist oder 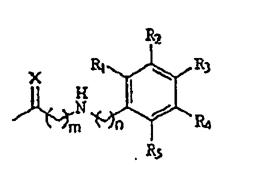 (wobei X Sauerstoff oder Schwefel ist, R₁, R₂, R₃, R₄ und R₅ unabhängig voneinander Wasserstoff, Halogen, Nitro, Amino, Hydroxy, C₁-C₄ niederes Alkylhydroxy, C₁-C₄ niederes Alkylamino, C₁-C₈ Alkyl, C₁-C₄ niederes Alkoxy oder C₁-C₄ niederes Alkyloxycarbonyl sind und m und n unabhängig voneinander eine ganze Zahl von 0, 1 oder 2 sind),
R₆, R₇, R₈ und R₉ unabhängig voneinander Wasserstoff, C₁₋₈ Alkyl oder (C₁-C₄) niederes Alkoxy sind und
Y ist -N=CHR' (wobei R' Wasserstoff ist, Benzyl, C₁₋₈ Alkyl oder (C₁-C₆) niederes Alkylamino), 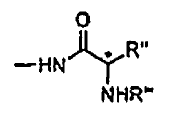 (wobei R" Wasserstoff ist, Benzyl, C₁-C₈ Alkyl oder C₁-C₆ niederes Alkylamino und R''' ist Wasserstoff, Benzyl, C₁-C₈ Alkyl oder eine Aminoschutzgruppe) oder 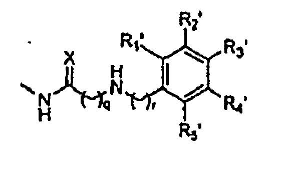 wobei X wie oben definiert ist,
R₁', R₂', R₃', R₄' und R₅' unabhängig voneinander Wasserstoff, Halogen, Nitro, Amino, Hydroxy, C₁-C₄ niederes Alkylhydroxy, C₁-C₄ niederes Alkylamino, C₁-C₈ Alkyl, C₁-C₄ niederes Alkoxy oder C₁-C₄ niederes Alkylcarboxy sind und q und r unabhängig voneinander eine ganze Zahl von 0, 1 oder 2 sind) oder deren pharmazeutisch verträgliches Salz.

2. Verfahren zur Herstellung einer Verbindung nach Anspruch 1, beinhaltend Reagieren einer Verbindung der Formel (a) mit einem -C=X-bildenden Reagens zum Herstellen einer Verbindung der Formel (b), Reagieren der Verbindung der Formel (b) mit einer Verbindung der Formel (c) zum Herstellen einer Verbindung nach Anspruch 1 und wahlweises Umwandeln derselben in ein Säureadditionssalz derselben 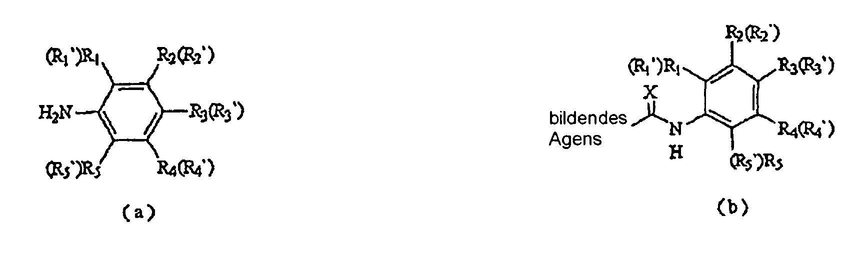 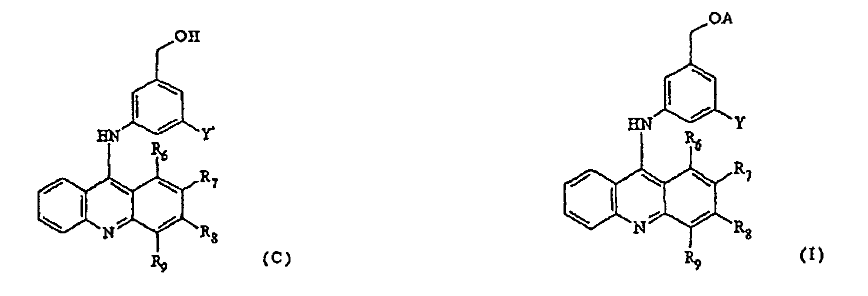 wobei Y' H oder -NH₂ ist und wobei die anderen Substituenten wie in Anspruch 1 definiert sind.

3. Verfahren zur Herstellung einer Verbindung nach Anspruch 1, beinhaltend Reagieren einer Verbindung der allgemeinen Formel (d) mit HCOR' in Gegenwart einer Base und eines organischen Lösungsmittels zum Herstellen einer Verbindung der Formel (I) 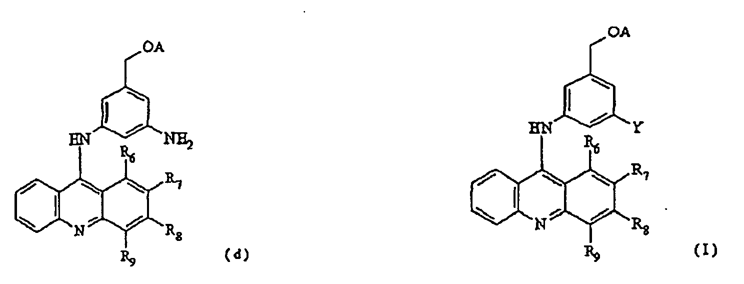 wobei die Substituenten wie in Anspruch 1 definiert sind.

4. Verfahren zur Herstellung einer Verbindung nach Anspruch 1, beinhaltend Reagieren einer Verbindung der allgemeinen Formel (d) mit 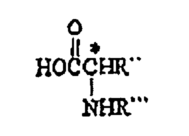 in Gegenwart einer Base und eines organischen Lösungsmittels zum Herstellen einer Verbindung der Formel (I) 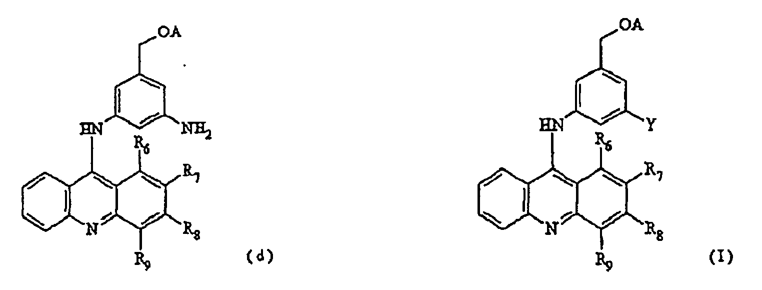 wobei die Substituenten wie in Anspruch 1 definiert sind.

## Revendications

1. Composé de formule générale (I) 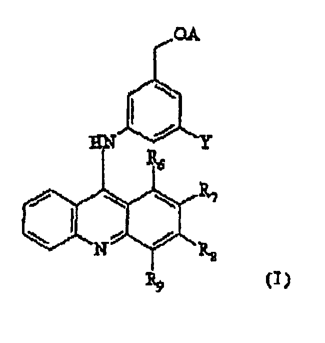 dans laquelle A est un hydrogène ou 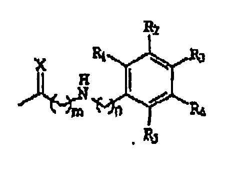 (dans laquelle X est un atome d'oxygène ou de soufre, R₁, R₂, R₃, R₄ et R₅ sont indépendamment de l'hydrogène, un halogène, un nitro, un amino, un hydroxy, un alkylhydroxy inférieur en C₁-C₄, un alkylamino inférieur en C₁-C₄, un alkyle en C₁-C₈, un alcoxy inférieur en C₁-C₄, ou un alkyloxycarbonyle inférieur en C₁-C₄, et m et n sont indépendamment un nombre entier de 0, 1 ou 2).
R₆, R₇, R₈, et R₉ sont indépendamment un hydrogène, un alkyle en C₁₋₈, ou un alcoxy inférieur en (C₁-C₄), et Y est un -N=CHR' (dans lequel R' est un hydrogène, un benzyle, un alkyle en C₁₋₈ d'alkylamino inférieur en (C₁-C₆)), 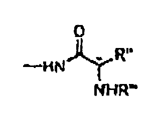 (dans laquelle R'' est un hydrogène, un benzyle, un alkyle en C₁-C₈, ou un alkylamino inférieur en C₁-C₆, et R''' est un hydrogène, un benzyle, un alkyle en C₁-C₈ ou un groupe de protection amino) ou 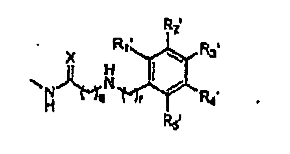 dans lequel X est comme défini ci-dessus,
R₁', R₂', R₃', R₄' et R₅' sont indépendamment de l'hydrogène, un halogène, un nitro, un amino, un hydroxy, un alkylhydroxy inférieur en C₁-C₄, un alkylamino inférieur en C₁-C₄, un alkyle en C₁-C₈, un alcoxy inférieur en C₁-C₄, ou un alkylcarboxy en C₁-C₄ et q et r sont indépendamment un nombre entier de 0, 1, ou 2) ou son sel pharmaceutiquement acceptable.

2. Procédé pour la préparation d'un composé selon la revendication 1, comprenant la réaction d'un composé de formule (a) avec un réactif fournissant -C=X pour donner un composé de formule (b), la réaction du composé de formule (b) avec un composé de formule (c) pour donner un composé selon la revendication 1, et éventuellement la conversion de celui-ci en un sel d'addition acide de celui-ci. 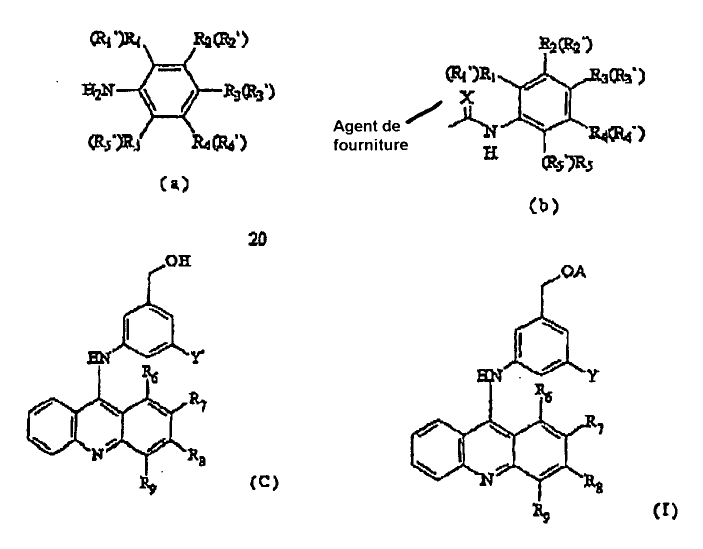 dans laquelle Y' est un H ou -NH₂, et les autres substituants sont comme définis dans la revendication 1.

3. Procédé pour la préparation d'un composé selon la revendication 1, comprenant la réaction d'un composé de formule générale (d) avec du HCOR' en présence d'une base et d'un solvant organique pour donner un composé de formule (I) 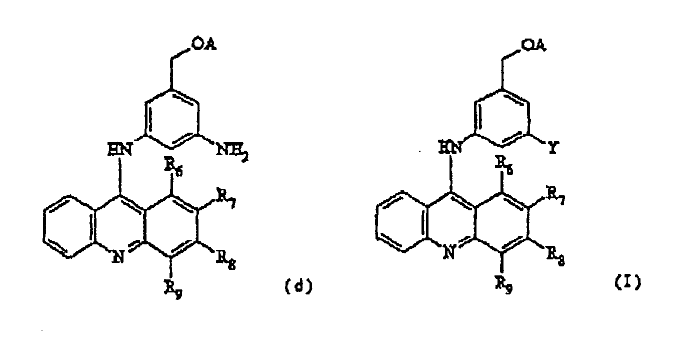 dans laquelle les substituants sont comme définis dans la revendication 1.

4. Procédé pour la préparation d'un composé selon la revendication 1, comprenant la réaction d'un composé de formule générale (d) avec 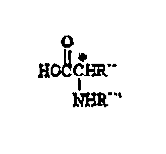 en présence d'une base et d'un solvant organique pour donner un composé de formule (I), 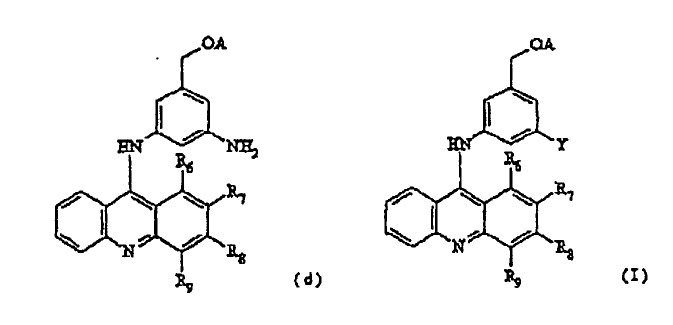 dans laquelle les substituants sont comme définis dans la revendication 1.
